# EUROPEAN PATENT SPECIFICATION

(11) **EP 2 747 830 B1**
(45) Date of publication and mention of the grant of the patent: **13.10.2021**
(21) Application number: 12825105.5
(22) Date of filing: 22.08.2012
(51) Int. Cl.: A61M 25/09, A61M 25/088, A61N 5/02, A61N 1/05, A61N 1/36, A61N 7/02, A61B 18/14, A61B 18/00, A61B 18/02, A61B 18/18

(54) **DEVICES FOR TREATING HYPERTENSION WITH ENERGY**
VORRICHTUNGEN ZUR BEHANDLUNG VON HYPERTONIE MIT ENERGIE
DISPOSITIFS DE TRAITEMENT DE L'HYPERTENSION AVEC DE L'ÉNERGIE

(30) Priority: 24.08.2011 US 201113217233
(43) Date of publication of application: 02.07.2014
(73) Proprietor: Heuser, Richard R., Phoenix, AZ 85016 (US)
(72) Inventor: Heuser, Richard R., Phoenix, AZ 85016 (US)
(74) Representative: Clark, Jane Anne
(86) International application number: PCT/US2012/051950
(87) International publication number: WO 2013/028812

(56) References cited:
- WO-A1-2009/097294
- WO-A1-2011/053757
- WO-A1-2011/112400
- WO-A2-2007/078997
- US-A1- 2006 276 852
- US-A1- 2007 129 720
- US-A1- 2009 192 485

## Description

### Technical Field

Embodiments herein relate to the field of hemodynamics, and more specifically to devices for the treatment of hypertension.

### Background

Hypertension is a cardiac chronic medical condition in which the systemic arterial blood pressure is elevated. Hypertension is classified as either primary (essential) hypertension or secondary hypertension, and about 90-95% of cases are categorized as "primary hypertension," the causes of which is poorly understood. The remaining 5-10% of cases are classified as secondary hypertension, and are caused by other conditions that affect the kidneys, arteries, heart, or endocrine system.

Persistent hypertension is a risk factor for stroke, myocardial infarction, heart failure and arterial aneurysm, and is a leading cause of chronic kidney failure, and even moderate elevation of arterial blood pressure leads to shortened life expectancy. Dietary and lifestyle changes and medication can improve blood pressure control and decrease the risk of associated health complications in many individuals, however these measures are ineffective or insufficient in some patients with hypertension.

US 2006/0276852 describes methods and apparatus for treating hypertension via a pulsed electric field, via a stimulation electric field, via localized drug delivery, via high frequency ultrasound, via thermal techniques, etc. The apparatus may comprise a catheter having a centring element with electrodes arranged near a proximal end of the centering element and the electrodes arranged near a distal end of the centering element.

US 2007/0129720 describes methods and apparatus for non-continuous circumferential treatment of a body lumen as shown in Figures 7A and 7B, the apparatus has multiple angularly off-set electrodes.

WO 2007/078997 describes methods and apparatus for pulsed electric field neuromodulation in which an intravascular catheter having one or more electrodes is configured for intra-to-extravascular placement across the wall of a patient's vessel into proximity with target neural fibers.

WO 2011/053757 describes methods and apparatus for treatment of hypertension through percutaneous ultrasound renal denervation in which an ultrasonic transducer is inserted into the renal artery by advancing the distal end of a catheter bearing the transducer into the renal artery.

The present invention is set out in the appended claims. Aspects, embodiments and examples disclosed herein which do not fall within the scope of the appended claims do not form part of the invention, and are merely provided for illustrative purposes.

### Brief Description of the Drawings

Embodiments will be readily understood by the following detailed description in conjunction with the accompanying drawings. Embodiments are illustrated by way of example and not by way of limitation in the figures of the accompanying drawings.

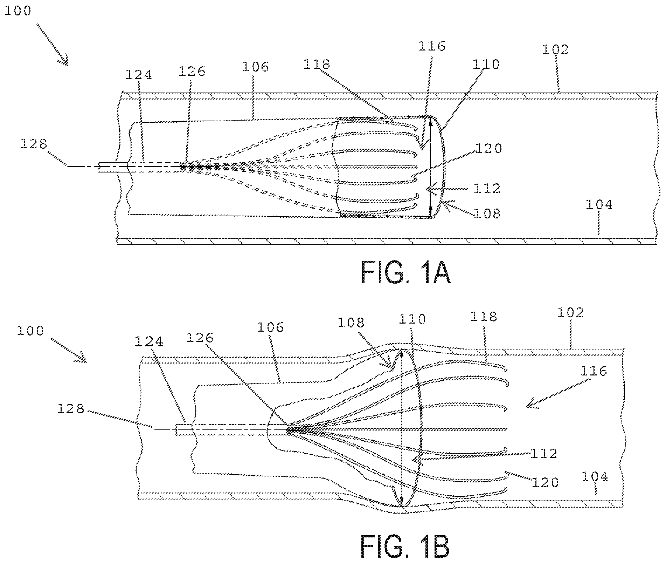

**Figures 1A** and **1B** illustrate an example of a device for delivering energy for a renal nerve, the device for delivering energy to a renal nerve, the device having a plurality of wires that may be moved between a closed position **(****Figure 1A****)** and an open position **(****Figure 1B****),** in accordance with various embodiments;

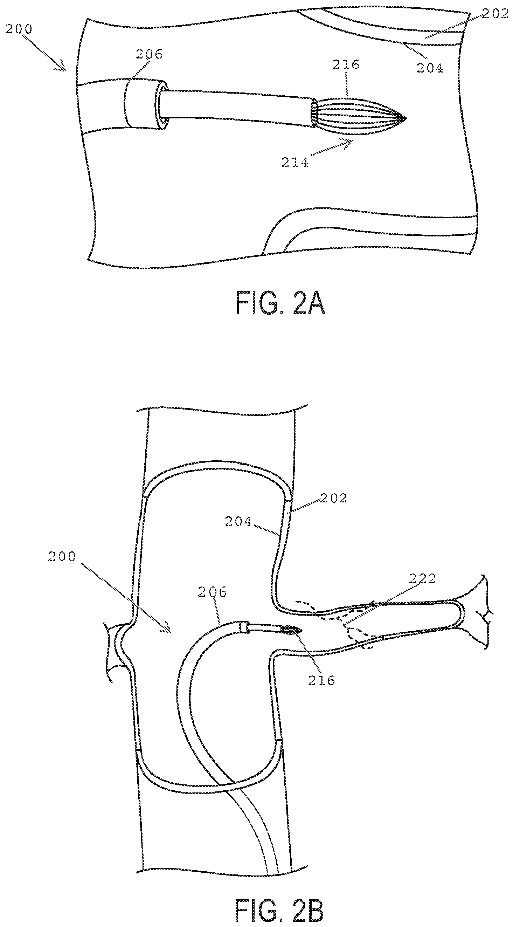

**Figures 2A** and **2B** illustrate another example of a device for delivering energy to a renal nerve, the device having a plurality of wires that may be coupled together at their distal ends, as illustrated in a close-up view **(****Figure 2A****)** and in a position adjacent a renal nerve **(****Figure 2B****),** in accordance with various embodiments; and

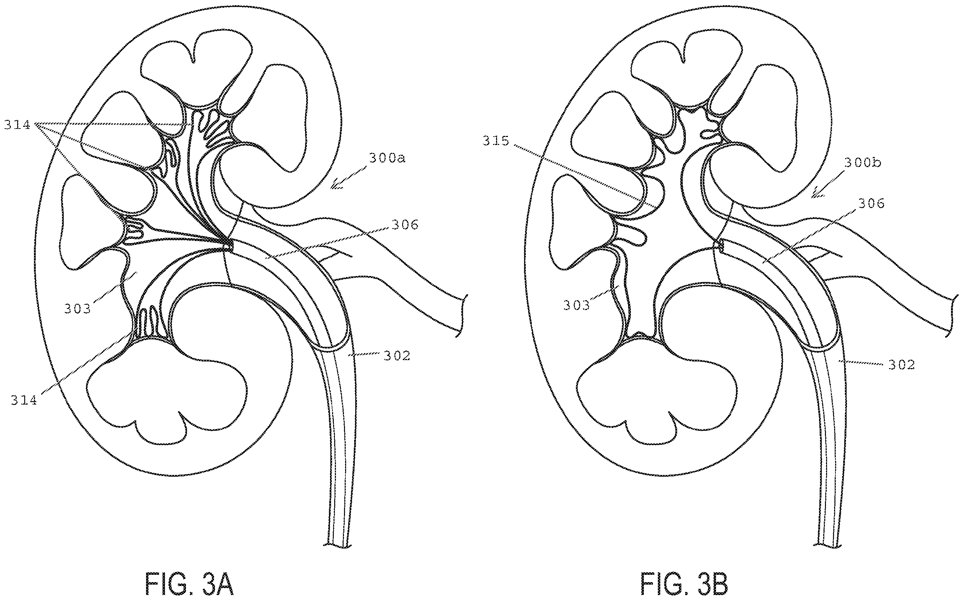

**Figures 3A** and **3B** illustrate two examples of a device for delivering energy to a renal nerve via the urinary collecting system and/or renal pelvis, the device having a plurality of fine wires or mesh elements **(****Figure 3A****)** or a single wire or mesh element **(****Figure 3B****)** configured to be moved between a closed position and an open position within the renal pelvis, in accordance with various embodiments.

### Detailed Description of Disclosed Embodiments

In the following detailed description, reference is made to the accompanying drawings which form a part hereof, and in which are shown by way of illustration embodiments that may be practiced. It is to be understood that other embodiments may be utilized and structural or logical changes may be made without departing from the of the following claims. Therefore, the following detailed description is not to be taken in a limiting sense, and the scope of embodiments is defined by the appended claims.

Various operations may be described as multiple discrete operations in turn, in a manner that may be helpful in understanding embodiments; however, the order of description should not be construed to imply that these operations are order dependent.

The description may use perspective-based descriptions such as up/down, back/front, and top/bottom. Such descriptions are merely used to facilitate the discussion and are not intended to restrict the application of disclosed embodiments.

The terms "coupled" and "connected," along with their derivatives, may be used. It should be understood that these terms are not intended as synonyms for each other. Rather, in particular embodiments, "connected" may be used to indicate that two or more elements are in direct physical or electrical contact with each other. "Coupled" may mean that two or more elements are in direct physical or electrical contact. However, "coupled" may also mean that two or more elements are not in direct contact with each other, but yet still cooperate or interact with each other.

For the purposes of the description, a phrase in the form "A/B" or in the form "A and/or B" means (A), (B), or (A and B). For the purposes of the description, a phrase in the form "at least one of A, B, and C" means (A), (B), (C), (A and B), (A and C), (B and C), or (A, B and C). For the purposes of the description, a phrase in the form "(A)B" means (B) or (AB) that is, A is an optional element.

The description may use the terms "embodiment" or "embodiments," which may each refer to one or more of the same or different embodiments. Furthermore, the terms "comprising," "including," "having," and the like, as used with respect to embodiments, are synonymous.

Embodiments herein provide systems for the treatment of hypertension. In various embodiments, devices are provided that may be used to treat hypertension in a subject by applying energy, such as radio frequency (RF), microwave, ultrasound, or cryo energy, to one or more renal sympathetic and/or parasympathetic nerves, thereby selectively denervating the renal sympathetic and/or parasympathetic nerves.

RF energy has been used to treat hypertension previously, but the precise location of the renal sympathetic and parasympathetic nerves is difficult to ascertain, and known methods of denervation can cause damage to surrounding tissues. The renal sympathetic and parasympathetic nerves are adjacent to and wrap generally around (e.g., in casings or layers) the renal artery in humans, and sympathetic denervation has previously been performed using a fairly rigid probe at the tip of a catheter. In such a procedure, an interventionist may perform an arteriogram to determine the location of the renal artery, and then may apply RF energy successively in several sites inside the renal artery (e.g., at 12 o'clock, 3 o'clock, 6 o'clock and 9 o'clock. RF energy may be transmitted through the vessel walls to damage and/or disable the adjacent renal nerves. Examples of this technique are described in "Catheter-Based Renal Denervation for Blood Pressure Reduction," Krishna Rocha-Singh, Volume: 17 of Cath Lab Digest (available at http://www.cathlabdigest.com/articles/Catheter-Based-Renal-Denervation-Blood¬Pressure-Reduction, last checked July 28, 2011) .

A problem with this technique is that it requires the interventionist to move the rigid probe (and particularly the energy-emitting tip) around the renal artery multiple times in order to apply energy to multiple locations. Generally, the more the interventionist is required to move around the instrument, the higher the risk of trauma to the vessel wall.

By contrast, in the methods and devices disclosed herein, instead of a rigid probe with a single energy source on the tip, an exemplary apparatus in accordance with the present disclosure may include a plurality of wires or one or more flexible mesh elements that are extendable from a catheter to expand and contact the inner wall of a conduit, such as a vascular or urinary conduit, at a plurality of points. Additionally, in various embodiments the disclosed methods and devices may be used not only in the renal arteries, but also in the renal veins and/or in the renal pelvis. As used herein, the term "conduit" encompasses arteries, veins, and portions of the urinary collection system, such as the renal pelvis and ureters.

In various embodiments, energy (e.g., RF, microwave, ultrasound, or cryo energy) may then be applied to the inner wall of the conduit at each of these contact points, thereby treating the adjacent nerves in multiple locations at once, without requiring the interventionist to move the apparatus around the conduit multiple times. In various embodiments, the device may be deployed in the renal artery, the renal vein, within a ureter, or within a renal capsule.

**Figures 1A** and **1B** illustrate an example of a device for delivering energy to a renal nerve, the device having a plurality of wires that may be moved between a closed position **(****Figure 1A****)** and an open position **(****Figure 1B****),** in accordance with various embodiments. Referring now to **Figures 1A** and **1B****,** an exemplary apparatus **100** in accordance with the present disclosure is shown in a vessel **102** with an inner wall **104.** In various embodiments, apparatus **100** may include a catheter **106.** In various embodiments, catheter **106** may include a distal end **108,** an opening **110** adjacent to distal end **108,** and a lumen **112** traversing the length of catheter **106** and terminating or being in communication with opening **110.** In some embodiments, distal end **108** of catheter **106** may be insertable to a position within vessel **102.**

In the embodiment shown in **Figures 1A** and **1B****,** distal end **108** of catheter **106** may be manipulable between a first configuration where opening **110** has a first diameter labeled **'A'** in **Figure1A****,** and a second configuration where opening **110** has a second diameter labeled **'B'** in **Figure 1B****,** although this is not required. In various embodiments, the second diameter **B** may be greater than the first diameter, as seen in **Figures 1A** and **1B****.**

In various embodiments apparatus **100** may include a plurality **114** of wires having a plurality **116** of distal ends. Various numbers of wires may be included. For example, in some embodiments, the plurality **114** of wires may include fourteen, sixteen, eighteen, or more wires.

In various embodiments, the plurality **114** of wires may be manipulable between a various positions. For instance, in some embodiments, plurality **114** of wires may be manipulated (e.g., advanced and retracted) between a position wherein the plurality **116** of distal ends are within lumen **112,** as shown in **Figure 1A****,** and another position wherein the plurality **116** of distal ends are extended out of lumen **112** through opening **110,** as shown in **Figure 1B**. In the embodiment illustrated in **Figures 1A** and **1B****,** each wire has a main portion **118** and an individual distal end **120.** In this illustrated embodiment, distal ends **120** may be separate from one another and not coupled together.

In various embodiments, plurality **114** of wires may be used to deliver sufficient energy to the renal nerve to cause denervation to occur. For example, in embodiments where radiofrequency energy is delivered via plurality **114** of wires, a temperature of about 55-70 degrees Centigrade may be applied for approximately 40 seconds is several (e.g., 3-10) areas of the nerve using approximately 5-10 watts, for example 8 watts, in specific, non-limiting embodiments. In particular embodiments, vascular access may be obtained using conventional means, such as a 6 French (2 mm) system.

**Figures 2A** and **2B** illustrate another example of a device for delivering energy to a renal nerve, the device having a plurality of wires that may be coupled together at their distal ends, as illustrated in a close-up view **(****Figure 2A****)** and in a position adjacent a renal nerve **(****Figure 2B****),** in accordance with various embodiments. Referring now to **Figures 2A** and **2B****,** the exemplary device **200** is shown in a vessel **202** with an inner wall **204.** In various embodiments, apparatus **200** may include an catheter **206.** As illustrated, device **200** may be insertable to a position within vessel **202,** and may be navigated through one or more bifurcations into renal artery **202a.** In the illustrated embodiment, a portion of a renal nerve **222** is shown adjacent renal artery **202a.**

In various embodiments, device **200** may include a plurality **214** of wires. Various numbers of wires may be included, for example, fourteen, sixteen, eighteen, or more wires. In the illustrated embodiment, the wires may be coupled together at their distal ends **220.** Regardless of whether the distal ends **120, 220** of the wires are formed or coupled together, in some embodiments, the wires may be biased away from one another so that when they are extended from the catheter **106, 206,** they expand to contact the inner walls **104, 204** of the vessel **102, 202** at multiple points at once.

The devices disclosed herein, including the examples illustrated in **Figures 1** and **2****,** may be used as follows to damage or disable nerves that are adjacent conduits, such as veins, arteries, the renal pelvis, or ureters, in order to treat hypertension. First, with reference to **Figures 2A** and **2B****,** in various embodiments, the catheter **206** of the device **200** (e.g., which may form a probe of about 0.035 inches in diameter in specific, non-limiting examples) may be positioned adjacent or within a vessel **202** or other conduit, such as one of the renal arteries **202a** as illustrated in **Figure 2B****.** In various embodiments, at lease a portion of renal artery **202a** may be adjacent and/or wrapped with renal nerves **222.** In various embodiments, once catheter **206** is in a position within vessel **202** adjacent renal nerves **222,** wires, such as the plurality **114** of wires illustrated in **Figures 1A** and **B** or the plurality **214** of wires shown in **Figures 2A** and **2B** may be extended out of the distal end of catheter **102, 202.** In various embodiments, once extended, because the plurality **114, 214** of wires are biased away from each other, they will expand to contact the inner walls **104, 204** of vessel **102, 202** at as many positions as there are wires.

In various embodiments, energy such as RF (e.g., about 80 degrees centigrade) may then be applied to the conduit wall through each wire (simultaneously or in various sequences) without having to move the wires or the apparatus. In various embodiments, the energy may be transmitted through the conduit wall to the renal nerves **222,** thereby damaging or disabling the renal nerves **222.**

In various embodiments, the wires such as the plurality **114** of wires shown in **Figures 1A** and **1B****,** and the plurality **214** of wires shown in **Figures 2A** and **2B** may be extended out of the catheter **206** to various lengths. For example, in some embodiments, the plurality **214** of wires may be extended approximately 30 cm from the catheter **206.** and may achieve apposition to large expanse of the inner wall **204** of vessel **202.** In various embodiments, energy may be applied along the entire length of the extended wires, thereby treating a large portion of vessel **202** (and disabling the adjacent renal nerves **222**) in a single operation. In various embodiments, the wires may then be withdrawn back into catheter **206** so that catheter **206** may be removed from the body atraumatically.

In other embodiments, the wires may be extended less distance from catheter **214,** about 6 cm for example, so as to avoid unnecessary trauma to the vessel **202** wall if there is not room to extend the wires further. In various embodiments, the wires may be long enough to give the interventionist considerable flexibility in how far the wires are extended out of the catheter **206.** In some instances, catheter **206** may be positioned at the orifice of the renal artery **202a** coming out of the aorta, so that just the wires extend into the renal artery **202a.**

Turning once again to **Figures 1A** and **1B****,** in some embodiments, each distal end **120** may terminate in an angled portion **124** which may form an angle relative to the rest of the wire. In various embodiments, these angles may be any angle between 0° and 180°, such as 30°, 45°, 90°, or 120°. In various embodiments, each angled portion **124** may be positioned so that it will not contact inner wall **104** of vessel **102,** thereby preventing trauma to vessel **102.**

In some embodiments, such as those illustrated in **Figures 1A** and **1B****,** device **100** may include an inner wire **126** which may forms an axis **128** extending through lumen **112.** In various embodiments, plurality **114** of wires may include a plurality of proximal ends each seamlessly bound to inner wire **126.** In some embodiments, plurality **116** of distal ends may be nominally biased away from axis **128.** In some embodiments, such as those shown in **Figures 1A, 1B****,** **2A,** and **2B****,** plurality **114** of wires may be curved. In other embodiments, plurality **114** of wires may be straight.

**Figures 3A** and **3B** illustrate two examples of a device for delivering energy to a renal nerve via the urinary collection system and/or renal pelvis, the device having a plurality of fine wires or mesh elements **(****Figure 3A****)** or a single wire or mesh element **(****Figure 3B****)** configured to be moved between a closed position and an open position within the renal pelvis, in accordance with various embodiments. Although these embodiments are described and illustrated as being used in the renal pelvis, one of skill in the art will appreciate that the same embodiments also may be used in the renal arteries or renal veins. Referring now to **Figures 3A** and **3B****,** the exemplary device **300a, 300b** is shown with catheter **306** in a portion of the ureter **302** adjacent the renal pelvis **303.** As illustrated, device **300a, 300b** may be insertable through the urinary collection system (e.g., urethera, bladder, and ureter) to a position adjacent renal pelvis **303.**

In various embodiments, device **300a, 300b** may include a plurality **314** of wires or fine mesh elements (e.g., as shown in **Figure 3A**), or a single wire or fine mesh element **315** (e.g., as shown in **Figure 3B**). Various numbers of wires or mesh elements **315** may be included, for example, one, two, three, four, five, six, or more wires or mesh elements.

In various embodiments, the plurality **314** of wires or fine mesh elements or single wire or fine mesh element **315** may be biased into an open position such that it may open (e.g., similar to a fishing net) when catheter **306** is retracted (or when the plurality **314** of wires or fine mesh elements or single wire or fine mesh element **315** is advanced from within the lumen of catheter **306**). In various embodiments, the wires or mesh have a high degree of flexibility, and may include a plurality of very compliant struts of wires, for example made from Nitinol™.

In various embodiments, device **300** may be used in the urinary collection system. In various embodiments, device **300** may be passed through the urethra of a male or female subject, into the bladder, and up into the ureter and renal pelvis. In various embodiments, after device **300** is passed from the narrow ureter into the wider renal pelvis, catheter **306** may be withdrawn to expose the plurality **314** of wires or fine mesh elements or single wire or fine mesh element **315.** In various embodiments, as the plurality **314** of wires or fine mesh elements or single wire or fine mesh element **315** opens, it may expand to fill all or a portion of the renal pelvis and may generally follow the contours therein.

In some embodiments, device **300** may be used within the upper portion of the ureter or within the renal pelvis, close to the neuroplexis of nerves innervating the renal system. In embodiments, device **300** may be used to apply either radiofrequency energy through a transducer or microwave energy or cryo energy. In particular specific, non-limiting embodiments, a microwave energy may be configured to achieve a temperature in the targeted nerve of about 60 to 75 degrees Centigrade. In other specific, non-limiting embodiments, cryo energy may be applied to lower the temperature of the targeted nerve to a predetermined temperature sufficient to cause denervation in whole or in part. In still other specific, non-limiting embodiments, ultrasound may be applied, either alone or in combination with radiofrequency, microwave, or cryo energy.

In some embodiments, any of devices disclosed herein may be used with commercially available known RF systems such as those manufactured by Olympus, Inc. In various embodiments, the pliability and/or flexibility of the disclosed devices allow use in many areas of the nervous system. This flexibility may be needed in various embodiments, since it is difficult to assess in a living patient where a given set of nerves of interest located. Furthermore, the pliability and/or flexibility of the devices disclosed herein allows precise treatment without damage to delicate vascular and renal tissues.

Although certain embodiments have been illustrated and described herein, it will be appreciated by those of ordinary skill in the art that a wide variety of alternate and/or equivalent embodiments or implementations calculated to achieve the same purposes may be substituted for the embodiments shown and described without departing from the scope of the following claims. Those with skill in the art will readily appreciate that embodiments may be implemented in a very wide variety of ways. This application is intended to cover any adaptations or variations of the embodiments discussed herein. Therefore, it is manifestly intended that embodiments be limited only by the claims.

## Claims

1. A device for denervating a renal nerve, the apparatus comprising:
a catheter (306) having a distal end insertable to a first position within a conduit adjacent to the renal nerve, an opening adjacent to the catheter distal end, and a lumen in communication with the opening; and
one or more flexible wires (314; 315), wherein the one or more flexible wires (314; 315) are manipulable between a first position wherein the one or more flexible wires (314; 315) are within the lumen, and a second position wherein the one or more flexible wires (314; 315) extend out of the lumen through the opening, the one or more flexible wires (314; 315) being configured to deliver energy to the renal nerve when the one or more flexible wires are in the second position, wherein the one or more flexible wires (314; 315) are configured to expand to fill all or a portion of the renal pelvis and generally follow the contours therein.

2. The device of claim 1, wherein the energy is radiofrequency, microwave, ultrasound or cryo energy.

3. The device of claim 1, wherein the one or more flexible wires (314) comprise a fine mesh or net (315).

4. The device of claim 1, wherein the one or more flexible wires have a plurality of wire distal ends.

5. The device of claim 4, wherein the one or more wire distal ends are separate from one another.

6. The device of claim 1, wherein the plurality of distal ends each terminates in an angled portion configured so as, in use, not to contact an inner wall of the conduit.

7. The device of claim 4, wherein the one or more wire distal ends are coupled together.

8. The device of claim 1, wherein the one or more flexible wires (314; 315) are biased in an open position and configured to expand when in the second position.

9. The device of claim 1, wherein the catheter (306) is an expandable catheter and
wherein the catheter distal end is manipulable between a first configuration where the opening has a first diameter and a second configuration where the opening has a second diameter which is greater than the first diameter.

10. The device of claim 1, further comprising an inner wire forming an axis extending through the lumen, wherein the one or more flexible wires (314) further includes one or more proximal ends seamlessly bonded to the inner wire.

11. The device of claim 6, further comprising an inner wire forming an axis extending through the lumen, wherein the one or more flexible wires (314) further includes one or more proximal ends seamlessly bonded to the inner wire, the plurality of distal ends being nominally biased away from the axis.

12. The device of claim 1, wherein the conduit is a renal artery, a renal vein, a ureter, or a renal pelvis.

13. The device of claim 1, wherein the renal nerve is a sympathetic or parasympathetic nerve.

## Patentansprüche

1. Vorrichtung zur Denervierung eines Nierennervs, wobei die Vorrichtung umfasst:
einen Katheter (306) mit einem distalen Ende, das in eine erste Position innerhalb einer Leitung angrenzend an den Nierennerv einführbar ist, einer Öffnung angrenzend an das distale Ende des Katheters und einem Lumen in Kommunikation mit der Öffnung und
ein oder mehrere flexible Drähte (314; 315), wobei der eine oder die mehreren flexiblen Drähte (314; 315) zwischen einer ersten Position, wobei der eine oder die mehreren flexiblen Drähte (314; 315) innerhalb des Lumens sind, und einer zweiten Position, wobei der eine oder die mehreren flexiblen Drähte (314; 315) sich aus dem Lumen durch die Öffnung erstrecken, manipulierbar sind, wobei der eine oder die mehreren flexiblen Drähte (314; 315) dazu konfiguriert sind, Energie an den Nierennerv abzugeben, wenn der eine oder die mehreren flexiblen Drähte in der zweiten Position sind, wobei der eine oder die mehreren flexiblen Drähte (314; 315) dazu konfiguriert sind, sich auszudehnen, um das gesamte oder einen Teil des Nierenbeckens auszufüllen und allgemein den Konturen darin zu folgen.

2. Vorrichtung nach Anspruch 1, wobei die Energie Radiofrequenz-, Mikrowellen-, Ultraschall- oder Kryoenergie ist.

3. Vorrichtung nach Anspruch 1, wobei der eine oder die mehreren flexiblen Drähte (314) ein feines Geflecht oder Netz (315) umfassen.

4. Vorrichtung nach Anspruch 1, wobei der eine oder die mehreren flexiblen Drähte eine Vielzahl von distalen Drahtenden aufweisen.

5. Vorrichtung nach Anspruch 4, wobei das eine oder die mehreren distalen Drahtenden voneinander getrennt sind.

6. Vorrichtung nach Anspruch 1, wobei die Vielzahl von distalen Enden jeweils in einem abgewinkelten Teil endet, der dazu konfiguriert ist, im Gebrauch eine Innenwand der Leitung nicht zu berühren.

7. Vorrichtung nach Anspruch 4, wobei das eine oder die mehreren distalen Drahtenden aneinander gekoppelt sind.

8. Vorrichtung nach Anspruch 1, wobei der eine oder die mehreren flexiblen Drähte (314; 315) in einer offenen Position vorgespannt sind und dazu konfiguriert sind, sich auszudehnen, wenn sie in der zweiten Position sind.

9. Vorrichtung nach Anspruch 1, wobei der Katheter (306) ein ausdehnbarer Katheter ist und wobei das distale Katheterende zwischen einer ersten Konfiguration, in der die Öffnung einen ersten Durchmesser aufweist, und einer zweiten Konfiguration, in der die Öffnung einen zweiten Durchmesser aufweist, der größer als der erste Durchmesser ist, manipulierbar ist.

10. Vorrichtung nach Anspruch 1, weiterhin umfassend einen inneren Draht, der eine Achse bildet, die sich durch das Lumen erstreckt, wobei der eine oder die mehreren flexiblen Drähte (314) weiterhin ein oder mehrere proximale Enden beinhalten, die nahtlos an den inneren Draht gebondet sind.

11. Vorrichtung nach Anspruch 6, weiterhin umfassend einen inneren Draht, der eine Achse bildet, die sich durch das Lumen erstreckt, wobei der eine oder die mehreren flexiblen Drähte (314) weiterhin ein oder mehrere proximale Enden beinhalten, die nahtlos an den inneren Draht gebondet sind, wobei die Vielzahl von distalen Enden nominal von der Achse weg vorgespannt sind.

12. Vorrichtung nach Anspruch 1, wobei die Leitung eine Nierenarterie, eine Nierenvene, ein Harnleiter oder ein Nierenbecken ist.

13. Vorrichtung nach Anspruch 1, wobei der Nierennerv ein sympathischer oder parasympathischer Nerv ist.

## Revendications

1. Dispositif servant à la dénervation d'un nerf à destinée rénale, l'appareil comportant :
un cathéter (306) ayant une extrémité distale en mesure d'être insérée jusque sur une première position à l'intérieur d'un conduit adjacent par rapport au nerf à destinée rénale, une ouverture adjacente par rapport à l'extrémité distale du cathéter, et une lumière en communication avec l'ouverture ; et
un ou plusieurs fils flexibles (314 ; 315), dans lequel lesdits un ou plusieurs fils flexibles (314 ; 315) sont en mesure d'être manipulés entre une première position dans laquelle lesdits un ou plusieurs fils flexibles (314 ; 315) se trouvent à l'intérieur de la lumière, et une deuxième position dans laquelle lesdits un ou plusieurs fils flexibles (314 ; 315) s'étendent hors de la lumière au travers de l'ouverture, lesdits un ou plusieurs fils flexibles (314 ; 315) étant configurés pour délivrer de l'énergie au nerf à destinée rénale quand lesdits un ou plusieurs fils flexibles sont dans la deuxième position, dans lequel lesdits un ou plusieurs fils flexibles (314 ; 315) sont configurés pour s'élargir pour remplir l'intégralité ou une partie du pelvis rénal et suivre généralement les contours dans celui-ci.

2. Dispositif selon la revendication 1, dans lequel l'énergie est une énergie de radiofréquence, une énergie de micro-ondes, une énergie ultrasonore ou une cryo-énergie.

3. Dispositif selon la revendication 1, dans lequel lesdits un ou plusieurs fils flexibles (314) comportent un filet ou une maille fine (315).

4. Dispositif selon la revendication 1, dans lequel lesdits un ou plusieurs fils flexibles ont une pluralité d'extrémités distales de fils.

5. Dispositif selon la revendication 4, dans lequel lesdites une ou plusieurs extrémités distales de fils sont séparées les unes des autres.

6. Dispositif selon la revendication 1, dans lequel chaque extrémité de la pluralité d'extrémités distales se termine en une partie inclinée configurée de manière à, lors de l'utilisation, ne pas entrer en contact avec une paroi intérieure du conduit.

7. Dispositif selon la revendication 4, dans lequel lesdites une ou plusieurs extrémités distales de fils sont couplées ensemble.

8. Dispositif selon la revendication 1, dans lequel lesdits un ou plusieurs fils flexibles (314 ; 315) sont sollicités dans une position ouverte et configurés pour s'élargir quand ils sont dans la deuxième position.

9. Dispositif selon la revendication 1, dans lequel le cathéter (306) est un cathéter expansible et dans lequel l'extrémité distale du cathéter est en mesure d'être manipulée entre une première configuration dans laquelle l'ouverture a un premier diamètre et une deuxième configuration dans laquelle l'ouverture a un deuxième diamètre qui est supérieur par rapport au premier diamètre.

10. Dispositif selon la revendication 1, comportant par ailleurs un fil intérieur formant un axe s'étendant au travers de la lumière, dans lequel lesdits un ou plusieurs fils flexibles (314) comprennent par ailleurs une ou plusieurs extrémités proximales reliées sans soudure au fil intérieur.

11. Dispositif selon la revendication 6, comportant par ailleurs un fil intérieur formant un axe s'étendant au travers de la lumière, dans lequel lesdits un ou plusieurs fils flexibles (314) comprennent par ailleurs une ou plusieurs extrémités proximales reliées sans soudure au fil intérieur, les extrémités distales de la pluralité d'extrémités distales étant essentiellement sollicitées à distance de l'axe.

12. Dispositif selon la revendication 1, dans lequel le conduit est une artère rénale, une veine rénale, un uretère, ou un pelvis rénal.

13. Dispositif selon la revendication 1, dans lequel le nerf à destinée rénale est un nerf sympathique ou parasympathique.
